# EUROPEAN PATENT APPLICATION

(11) **EP 4 721 714 A1**
(43) Date of publication of application: **08.04.2026**
(21) Application number: 24814906.4
(22) Date of filing: 04.03.2024
(51) Int. Cl.: A61F 13/15, A61F 13/49, A61F 13/472, A61F 13/496

(54) **INDIVIDUALLY PACKAGED ABSORBENT ARTICLE**

(30) Priority: 29.05.2023 JP 2023087362
(71) Applicant: DAIO PAPER CORPORATION, Shikokuchuo-shi Ehime 799-0492 (JP)
(72) Inventor: TAKEZAWA, Hiromi, Sakura-shi, Tochigi 329-1411 (JP)
(74) Representative: Meissner Bolte Partnerschaft mbB
(86) International application number: PCT/JP2024/008055
(87) International publication number: WO 2024/247419

(57) **Abstract**

An individually packaged absorbent article in which an absorbent article is enclosed in a package is provided. The absorbent article is configured to form a panty shape by including a waist portion; and a crotch portion in which an absorber is disposed. The absorbent article has an L* value in an CIE 1976 L*a*b* color space of 40 or less when viewed from a non-skin-facing side, the non-skin-facing side being a side opposite to a skin-facing side of the absorbent article that faces a human body. The package has the L* value in the CIE 1976 L*a*b* color space of 50 or less when viewed from an outside.

## Description

### TECHNICAL FIELD

The present invention relates to an individually packaged absorbent article.

### BACKGROUND ART

In recent years, panty-type absorbent articles such as menstrual napkins are known (see, for example, Patent Document 1). Panty-type absorbent articles can be worn like panties because they include an absorber but have a panty shape as a whole. Such absorbent articles may be distributed as individually packaged absorbent articles enclosed in a package for reasons such as ensuring cleanliness during storage and enhancing convenience during transportation.

### RELATED ART DOCUMENTS

### PATENT DOCUMENTS

Patent Document 1: Japanese Laid-Open Patent Application No. 2016-73511

### SUMMARY OF THE INVENTION

### PROBLEMS TO BE SOLVED BY THE INVENTION

When an absorbent article is carried in a package, it is preferable that the absorbent article is not visible from the outside. When an absorbent article is disposed of after use, it is sometimes disposed in a package. In this case, it is not preferable that menstrual blood is visible from the outside.

The present invention has been made in view of the above issues, and provides an individually packaged absorbent article in which the absorbent article is individually enclosed in a package, menstrual blood is not appreciably seen through from the outside, and the absorbent article is not appreciably seen from the outside.

### MEANS FOR SOLVING THE PROBLEMS

In order to achieve the above object, the present invention provides:
an individually packaged absorbent article in which an absorbent article is enclosed in a package, wherein
the absorbent article is configured to form a panty shape by including
   a waist portion; and
   a crotch portion in which an absorber is disposed,
the absorbent article has an L* value in an CIE 1976 L*a*b* color space of 40 or less when viewed from a non-skin-facing side, the non-skin-facing side being a side opposite to a skin-facing side of the absorbent article that faces a human body, and
the package has the L* value in the CIE 1976 L*a*b* color space of 50 or less when viewed from the outside.

In the present invention configured as described above, the L* value in the CIE 1976 L*a*b* color space of the absorbent article when viewed from the non-skin-facing side of the absorbent article arranged opposite to the skin-facing side facing the human body is 40 or less, which makes it difficult for blood to be visible from the outside of the absorbent article after use. In addition, although the L* value when the absorbent article is viewed from the non-skin-facing side is 40 or less, the L* value when the absorbent article is viewed from the outside of the package is 50 or less, and thus the absorbent article becomes less visible from the outside when the absorbent article is enclosed in the package.

The color of the package in a state that the absorbent article is not enclosed in the package may be the same color as the color of the absorbent article when viewed from the non-skin-facing side in a state that the absorbent article is not enclosed in the package. In such a configuration, the absorbent article becomes less visible from the outside when the absorbent article is enclosed in the package.

In addition, the absorbent article may have a configuration in which the color of the absorbent article when viewed from the non-skin-facing side and the color of the package when viewed from the outside are black or gray. In such a configuration, the absorbent article becomes further less visible from the outside when the absorbent article is enclosed in the package.

In addition, the absorbent article may have the L* value in the CIE 1976 L*a*b* color space, when viewed from the skin-facing side, of 40 or less in any area of the absorbent article, and the package may have the L* value in the CIE 1976 L*a*b* color space, when viewed from the outside, of 50 or less in any area of the package. In such a configuration, blood becomes less visible from the outside of the absorbent article after use in any area of the absorbent article, and the absorbent article becomes less visible from the outside in any area of the package when the absorbent article is enclosed in the package.

### ADVANTAGEOUS EFFECTS OF THE INVENTION

According to the present invention, in an individually packaged absorbent article in which an absorbent article is individually enclosed in a package, menstrual blood is not appreciably visible from the outside and the absorbent article is not appreciably visible from the outside.

### BRIEF DESCRIPTION OF THE DRAWINGS

[FIG. 1] FIG. 1 is a diagram illustrating an embodiment of an individually packaged absorbent article of the present invention.
[FIG. 2] FIG. 2 is an external perspective view of a pair of menstrual panties as illustrated in FIG. 1.
[FIG. 3] FIG. 3 is a diagram illustrating a skin-facing side of the menstrual panties as illustrated in FIG. 2.
[FIG. 4] FIG. 4 is a diagram illustrating a cross-sectional view taken along a line A-A of FIG. 3.
[FIG. 5] FIG. 5 is a diagram illustrating a state in which the pair of menstrual panties as illustrated in FIG. 2 is worn.
[FIG. 6] FIG. 6 is a diagram illustrating a non-skin-facing side after absorption of menstrual blood according to Comparative Example 1.
[FIG. 7] FIG. 7 is a diagram illustrating the non-skin-facing side after absorption of menstrual blood according to Example 1.
[FIG. 8] FIG. 8 is a diagram illustrating the menstrual panties as illustrated in FIG. 2, in a state in which three-dimensional gather portions protrude from an outer body.
[FIG. 9] FIG. 9 is diagrams illustrating an exemplary structure of the package illustrated in FIG. 1. FIG. 9(a) is a diagram illustrating a plan view of the package. FIG. 9(b) is a diagram illustrating a view of the package seen from a direction of an arrow B illustrated in FIG. 9(a) with the package opened.
[FIG. 10] FIG. 10 is a diagram illustrating a state in which the pair of menstrual panties is enclosed in a package according to Comparative Example 2.
[FIG. 11] FIG. 11 is a diagram illustrating a state in which the pair of menstrual panties is enclosed in a package according to Example 4.

### DETAILED DESCRIPTION OF THE INVENTION

Embodiments of the present invention will be described in the following with reference to the drawings. In each of the drawings, unless otherwise explained, the same reference numerals may be assigned to the same or corresponding structures and the description thereof may be omitted. In the direction of orthogonal, lateral, and the like, deviations to the extent that they do not impair the action and effect of the embodiment are permitted. The shape of the corners is not limited to a right angle, but may be rounded in a bow shape. The term "orthogonal" may include "substantially orthogonal". In this specification, a front-rear direction of the menstrual panties 1 is D1, and a width direction of the menstrual panties 1 orthogonal to the front-rear direction is D2. The front-rear direction corresponds to the front-rear direction of a human body wearing the menstrual panties 1, and the width direction corresponds to a left-right direction of the human body wearing the menstrual panties 1. In this specification, a front side of the menstrual panties 1 in the front-rear direction is defined as a ventral side or the front, and a rear side of the menstrual panties 1 in the front-rear direction is defined as a dorsal side or the rear. In this specification, the side of the menstrual panties 1 facing the human body is defined as a skin-facing side, and the side of the menstrual panties 1 opposite to the skin-facing side is defined as a non-skin-facing side.

### <Overall structure>

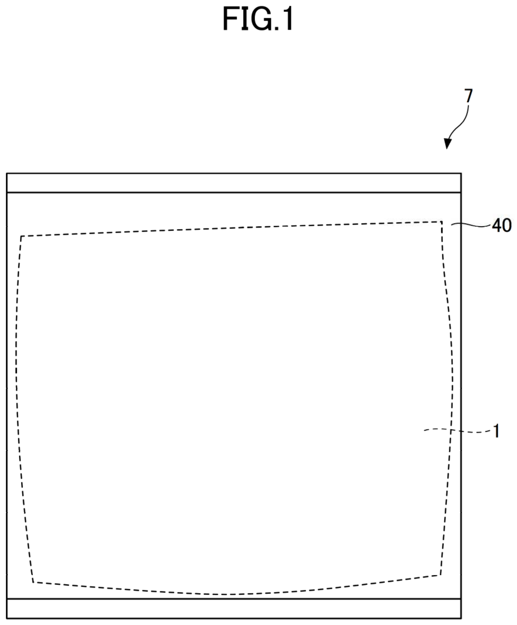

FIG. 1 is a diagram illustrating an embodiment of an individually packaged absorbent article according to the present invention.

As illustrated in FIG. 1, the present embodiment is an individually packaged absorbent article 7 in which a pair of menstrual panties 1 as an example of the absorbent article is enclosed in a package 40. The menstrual panties 1 are folded according to the shape of the package 40 and enclosed in the package 40.

### <Menstrual panties 1>

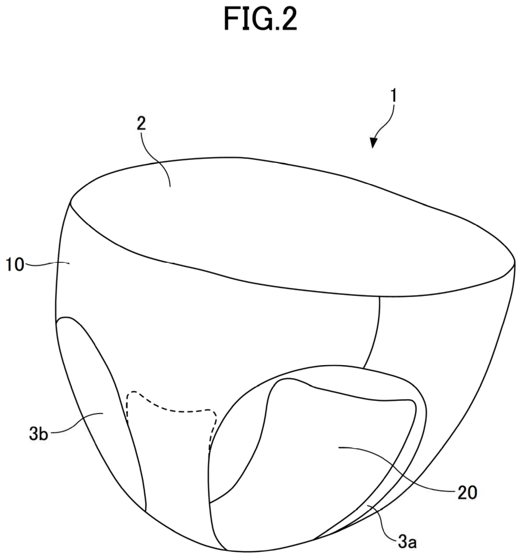

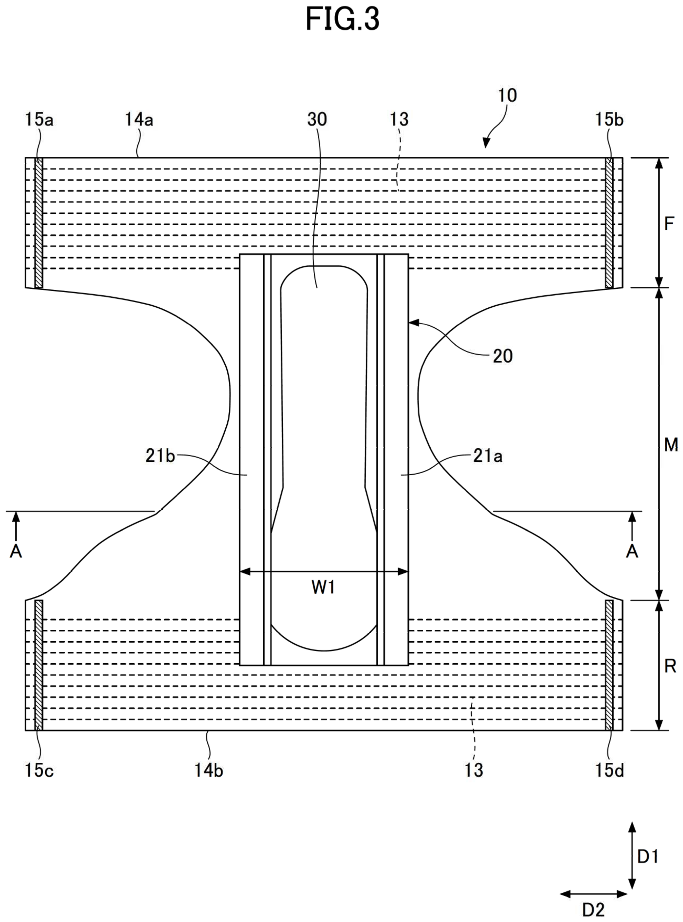

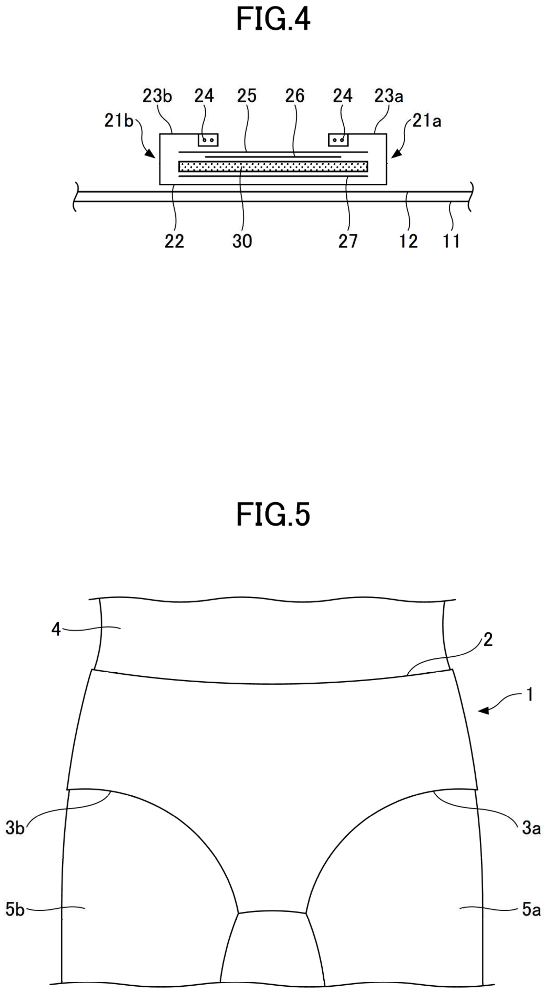

FIG. 2 is an external perspective view of the pair of menstrual panties 1 as illustrated in FIG. 1. FIG. 3 is a diagram illustrating the skin-facing side of the menstrual panties 1 as illustrated in FIG. 2, and is a plan view in an expanded state. FIG. 4 is a diagram illustrating a cross-sectional view taken along a line A-A of FIG. 3. FIG. 5 is a diagram illustrating a state in which the pair of menstrual panties 1 as illustrated in FIG. 2 is worn.

As illustrated in FIG. 2, the menstrual panties 1 of the present embodiment includes an outer body 10 and an inner body 20. The inner body 20 is arranged on one surface of the outer body 10. When the menstrual panties 1 are expanded, a front area F, a middle area M, and a rear area R are connected in the front-rear direction D1 in this order. The front area F covers the lower abdomen when the menstrual panties 1 are worn. The rear area R covers the lower waist on the rear side when the menstrual panties 1 are worn. The front area F and the rear area R constitute an example of a waist portion in the present invention. The middle area M constitutes an example of a crotch portion in the present invention, and is arranged between the front area F and the rear area R.

### <Outer body 10>

The outer body 10 is disposed over the front area F, the middle area M, and the rear area R. In the front area F and the rear area R, the outer body 10 has a belt-like shape having the width direction D2 as a longitudinal direction. The lengths in the width direction D2 of the outer body 10 in the front area F and the rear area R may be the same. In the middle area M, the outer body 10 is constricted in the width direction D2 toward the center such that leg openings 3a are formed when the menstrual panties 1 are worn.

The outer body 10 is composed of two sheets, an outer layer sheet 11 and an inner layer sheet 12, which are laminated together such that the outer layer sheet 11 is on the non-skin-facing side and the inner layer sheet 12 is on the inner body 20 side, and bonded together with an adhesive such as a hot-melt adhesive. The outer layer sheet 11 and the inner layer sheet 12 may only be partially bonded together with an adhesive. The outer layer sheet 11 may be folded inward at the end of the front area F side in the front-rear direction D1 of the menstrual panties 1.

The outer layer sheet 11 and the inner layer sheet 12 are not particularly limited as long as they are in the form of sheets, however, it is preferable to use nonwoven fabrics. In the case of nonwoven fabrics, raw material fibers are not particularly limited. For example, synthetic fibers such as olefinic fibers including polyethylene and polypropylene, synthetic fibers such as polyester and polyamide, recycled fibers such as rayon and cupra, natural fibers such as cotton, or mixed fibers or composite fibers containing two or more kinds of these may be used. Furthermore, the nonwoven fabric may be manufactured by any processing.

A plurality of elastic threads 13, which are an example of a stretchable member, are sandwiched between the outer layer sheet 11 and the inner layer sheet 12 in the front area F and the rear area R. The plurality of elastic threads 13 extend between both ends in the width direction D2 of the outer body 10 with the direction of expansion and contraction in the width direction D2, and are fixed between the outer layer sheet 11 and the inner layer sheet 12 in a state of being parallel to each other in the front-rear direction D1 with a space between them. The elastic threads 13 may be provided throughout the front area F and the rear area R, or may be provided only in a part of a predetermined width from an end 14a of the menstrual panties 1 in the front area F. Similarly, the elastic threads 13 may be provided only in a part of a predetermined width from an end 14b of the menstrual panties 1 in the rear area R. In addition, the elastic threads 13 may be provided partially throughout the front area F and the rear area R in the front-rear direction D1. The elastic threads 13 may be fixed to the outer layer sheet 11 and the inner layer sheet 12 by hot melt bonding, heat sealing, ultrasonic bonding, or the like. It is not necessary that all of the elastic threads 13 have the same thickness and elongation ratio. For example, the thickness and elongation ratio may be different between the end 14a, 14b sides of the menstrual panties 1 and the middle area M side.

Generally used styrene-based rubber, olefin-based rubber, urethane-based rubber, ester-based rubber, polyurethane, polyethylene, polystyrene, styrene-butadiene, silicone, polyester, and the like, can be used as the material included in the elastic threads 13.

Thus, in the present embodiment, a plurality of elastic threads 13 are sandwiched between the outer layer sheet 11 and the inner layer sheet 12. Thus, the stretchability in the width direction D2 of the menstrual panties 1 in the front area F and the rear area R can be secured by the elastic threads 13 sandwiched between the outer layer sheet 11 and the inner layer sheet 12. As the stretchable member, a stretchable film may be used instead of the elastic threads 13.

The outer body 10 includes bonding portions 15a and 15b at the ends, respectively, in the width direction D2 of the front area F. Also, in the rear area R, the outer body 10 includes bonding portions 15c and 15d at the ends, respectively, in the width direction D2. The bonding portion 15a of the front area F and the bonding portion 15c of the rear area R are bonded together by heat or the like, and the bonding portion 15b of the front area F and the bonding portion 15d of the rear area R are bonded together by heat or the like. Thus, as illustrated in FIG. 2, a waist opening 2 and the leg openings 3a and 3b are formed, respectively, and the outer body 10 forms a pair of panty-shaped menstrual panties 1.

In the present embodiment, although the outer body 10 is arranged over the front area F, the middle area M, and the rear area R, one of the outer layer sheet 11 or the inner layer sheet 12 may be divided into two in the front-rear direction D1. In addition, both of the outer layer sheet 11 and the inner layer sheet 12 may be divided into two in the front-rear direction D1. In view of appearance, it is most preferable that the outer body 10 is arranged over the front area F, the middle area M, and the rear area R. In view of manufacturing cost, it is preferable that both of the outer layer sheet 11 and the inner layer sheet 12 are divided into two in the outer body 10. In this case, when at least one of the outer layer sheet 11 or the inner layer sheet 12 is arranged over the front area F, the middle area M, and the rear area R, the waist portion of the present invention includes the outer body 10, and the crotch portion of the present invention includes the outer body 10 and the inner body 20. In a structure in which both of the outer layer sheet 11 and the inner layer sheet 12 are divided into two in the front-rear direction D1, the waist portion of the present invention includes the outer body 10, and the crotch portion of the present invention includes the inner body 20.

### <Inner body 20>

The inner body 20 is arranged over a part of the front area F and the rear area R as well as the middle area M, on the surface on the skin-facing side of the outer body 10 in a panty shape. In the inner body 20, a length W1 in the width direction D2 is the same as or shorter than the part where the length of the width direction D2 is shortest due to the constriction of the middle area M of the outer body 10. As a result, the inner body 20 includes an area that extends along the leg openings 3a and 3b when the outer body 10 is in the panty shape. The inner body 20 includes a gather sheet 22, a waterproof sheet 27, a second sheet 26, a top sheet 25, and an absorber 30.

The gather sheet 22 is arranged at the extreme of the non-skin-facing side of the inner body 20. For example, a nonwoven fabric is used for the gather sheet 22. Specifically, as the nonwoven fabric, a spunbonded nonwoven fabric, a meltblown nonwoven fabric, a spunlace nonwoven fabric, a thermally bonded (air-through) nonwoven fabric, a needle-punched nonwoven fabric, point bond nonwoven fabric, a laminated nonwoven fabric (spunbonded + spunbonded + spunbonded (SSS) nonwoven fabric in which the same or similar nonwoven fabric layers are laminated), and the like may be used.

The length of the gather sheet 22 in the front-rear direction D1 is the same as the length of the inner body 20, and both ends in the width direction D2 are folded back to a center side in the width direction D2 to form folded portions 23a and 23b. The folded portions 23a and 23b are arranged to partially overlap with the absorber 30. The gather sheet 22 is bonded to the inner layer sheet 12 by an adhesive over the entire length in the front-rear direction D1 in areas other than the folded portions 23a and 23b. Therefore, the folded portions 23a and 23b can stand up toward the inside of the panty shape of the outer body 10. A stretchable member 24 is attached to tips of the folded portions 23a and 23b so as to expand and contract in the front-rear direction D1. As a result, the folded portions 23a and 23b are included in three-dimensional gather portions 21a and 21b whose tips are contracted to be extendable. The three-dimensional gather portions 21a and 21b can stand up by utilizing a contraction force of the stretchable member 24. At this time, the length W1 of the inner body 20 in the width direction D2 is the same or shorter than the portion of the outer body 10 where the length in the width direction D2 is shortest due to the constriction of the middle area M. Therefore, the three-dimensional gather portions 21a and 21b stand up toward the inside of the panty shape of the outer body 10 in the area extending along both sides of the absorber 30 in the width direction D2. When the three-dimensional gather portions 21a and 21b stand up, the three-dimensional gather portions 21a and 21b can be brought into close contact with the body surface of the human body. As a result, the three-dimensional gather portions 21a and 21b have a function of preventing the lateral leakage of blood or the like traveling laterally on the top sheet 25.

As the stretchable member 24, materials such as a styrene-based rubber, an olefin-based rubber, a urethane-based rubber, an ester-based rubber, polyurethane, polyethylene, polystyrene, styrene-butadiene, silicone, polyester, and the like, which are normally used can be used.

As described above, in the present embodiment, the inner body 20 includes three-dimensional gather portions 21a and 21b which stand up inward of the panties in the area extending along both sides of the absorber 30 in the width direction D2. Thus, the leakage of blood or the like can be prevented, and it is not necessary to arrange thread rubber or the like in an area extending along the leg openings 3a and 3b for the leakage prevention, such that deterioration in the appearance and in the feeling of underwear can be avoided.

The waterproof sheet 27 is a waterblocking sheet which does not allow liquid such as blood to pass through, and is laminated and adhered on the skin-facing side surface of the gather sheet 22 by an adhesive or the like. As the waterproof sheet 27, for example, a microporous sheet obtained by kneading an inorganic filler in an olefinic resin such as polyethylene or polypropylene, molding the sheet, and then stretching it in a uniaxial or biaxial direction can be suitably used. In addition, as the waterproof sheet 27, a nonwoven fabric having enhanced waterproof property can be used as a base material. The waterproof sheet 27 preferably extends more widely than the absorber 30 at least in the front-rear direction D1 and the width direction D2.

In the present embodiment, the three-dimensional gather portions 21a and 21b are formed of one gather sheet 22. However, each of the three-dimensional gather portions 21a and 21b may be formed of one gather sheet. In this case, the two gather sheets are arranged on both sides of the inner body 20 in the width direction D2, and are bonded to the outer body 10 from the upper side of the top sheet 25. In this case also, the waterproof sheet 27 is arranged on the side opposite to the top sheet 25 via the absorber 30.

In the present embodiment, the three-dimensional gather portions 21a and 21b include the folded portions 23a and 23b formed by folding the ends in the width direction D2 of the gather sheet 22 toward the center in the width direction D2. However, the folded portions may be formed by folding the ends in the width direction D2 of the gather sheet 22 toward the outside in the width direction D2. In this case, the folded portions may be made narrower than the crotch width of the outer body 10 to prevent the three-dimensional gather portions 21a and 21b from protruding from the outer body 10.

The absorber 30 is disposed between the waterproof sheet 27 and, the top sheet 25 and the second sheet 26. The absorber 30 is not limited as long as it is a material capable of absorbing and retaining body fluids, but preferably includes a cotton-like pulp and a water-absorbing polymer. As the water-absorbing polymer, a highly water-absorbing polymer granular powder (superabsorbent polymer (SAP)), a highly water-absorbing polymer fiber (superabsorbent fiber (SAF)), or a combination of these can be used. Examples of the pulp include chemical pulp obtained from wood, cellulose fibers such as dissolved pulp, and artificial cellulose fibers such as rayon and acetate. The pulp may be hardwood pulp obtained from hardwood wood, softwood pulp obtained from softwood, or a mixture of these. The pulp may be recycled pulp recycled from used pulp.

The thickness of the absorber 30 may be 1.0 to 8.0 mm. The absorber 30 may have a structure in which an area corresponding to the position of the body fluid outlet (body-fluid-outlet corresponding area) or an area facing the grooves of the buttocks behind the body-fluid-outlet corresponding area is protruded.

The top sheet 25 and the second sheet 26 are laminated together and are disposed on the side opposite to the waterproof sheet 27 via the absorber 30, such that the second sheet 26 is disposed on the absorber 30 side.

For the top sheet 25 and the second sheet 26, a porous or non-porous nonwoven fabric or a porous plastic sheet is suitably used. As the material fibers included in the nonwoven fabric, for example, olefins such as polyethylene and polypropylene, synthetic fibers such as polyester and polyamide, recycled fibers such as rayon and cupra, and blended fibers of these, and natural fibers such as cotton can be used alone or in combination of two or more kinds. It is also possible to arrange only the top sheet 25 on the absorber 30 without arranging the second sheet 26.

As illustrated in FIG. 5, the menstrual panties 1 formed as described above are worn on a trunk 4 through the waist opening 2 and the legs 5a and 5b inserted through the leg openings 3a and 3b.

In a structure in which both the outer layer sheet 11 and the inner layer sheet 12 of the outer body 10 are divided into two, the outer body 10 may form the front area F and the rear area R, and the middle area M may be formed only by the inner body 20. In this case, a part of the inner body 20 extends to a part of the front area F and the rear area R, and the outer body 10 and the inner body 20 are bonded together. In such a structure, the inner body 20 may be laminated together with a cover sheet on the non-skin-facing side surface of the waterproof sheet 27.

### <Color of menstrual panties 1>

The color of menstrual panties 1 formed as described above will be described in the following.

Panty-type absorbent articles are often formed entirely in light colors such as white or pink. Therefore, after use, blood tends to be visible from the outside, which is the non-skin-facing side, and even though they are panty-type, they give the feeling of wearing diapers, and the appearance is poor.

Therefore, the color of the outer body 10 and the inner body 20 of menstrual panties 1 was changed, and the change in color appearance before and after the absorption of the menstrual blood by the absorber 30 was examined.

As described in the following, the L* value, a* value, and b* value in the CIE 1976 L*a*b* color space were measured before and after the absorption of the menstrual blood by using three menstrual panties of Examples 1 and 2 and Comparative Example 1. The values on the skin-facing side are the values of the inner body of the menstrual panties when viewed from the inside of the menstrual panties, and the values on the non-skin-facing side are the values of the outer body of the menstrual panties when viewed from the outside of the menstrual panties.

### (A) Example 1

Thickness of menstrual blood absorbing portion: 2.9 mm
Absorption amount of absorber: 920 g
Skin-facing side: white, L* value = 91.53, a* value = 0.63, b* value = 0.12
Non-skin-facing side: black, L* value = 19.19, a* value = 0.10, b* value = 1.81

### (B) Example 2

Thickness of menstrual blood absorbing portion: 3.2 mm
Absorption amount of absorber: 1,070 g
Skin-facing side: white, L* value = 91.53, a* value = 0.63, b* value = 0.12
Non-skin-facing side: dark gray, L* value = 37.70, a* value = 1.01, b* value = 0.32

### (C) Comparative Example 1

Thickness of menstrual blood absorbing portion: 2.9 mm
Absorption amount of absorber: 960 g
Skin-facing side: white, L* value = 91.53, a* value = 0.63, b* value = 0.12
Non-skin-facing side: white, L* value = 95.53, a* value = 0.49, b* value = 1.21

**[Table 1]**

| | | EXAMPLE 1 | EXAMPLE 2 | COMPARATIVE EXAMPLE 1 |
|---|---|---|---|---|
| BEFORE MENSTRUAL BLOOD ABSORPTION | SKIN-FACING SIDE | WHITE | WHITE | WHITE |
| | | L*=91.53 | L*=91.53 | L*=91.53 |
| | | a*=-0.63 | a*=-0.63 | a*=-0.63 |
| | | b*=0.12 | b*=0.12 | b*=0.12 |
| | NON-SKIN-FACING SIDE | BLACK | DARK GRAY | WHITE |
| | | L*=19.19 | L*=37.70 | L*=95.53 |
| | | a*=0.10 | a*=1.01 | a*=-0.49 |
| | | b*=1.81 | b*=0.32 | b*=1.21 |
| AFTER MENSTRUAL BLOOD ABSORPTION | SKIN-FACING SIDE | L*=28.47 | L*=29.57 | L*=25.50 |
| | | a*=39.34 | a*=38.48 | a*=34.40 |
| | | b*=15.45 | b*=15.35 | b*=14.69 |
| | NON-SKIN-FACING SIDE | L*=17.74 | L*=37.25 | L*=79.46 |
| | | a*=0.97 | a*=1.39 | a*=1.39 |
| | | b*=1.71 | b*=1.33 | b*=-1.57 |

Then, as illustrated in the above table, in Comparative Example 1, the L* value, which indicates the lightness of the color on the non-skin-facing side, was 95.53 before the menstrual blood absorption and changed significantly to 79.46 after the menstrual blood absorption. That is, the color seen from the non-skin-facing side changes from the white color before menstrual blood absorption to a dark color. This is because since the color seen from the non-skin-facing side before menstrual blood absorption is white, the blood of the menstrual blood is visible from the outside of the outer body of the menstrual panties, and thus leading the color of the non-skin-facing side to change to the dark color.

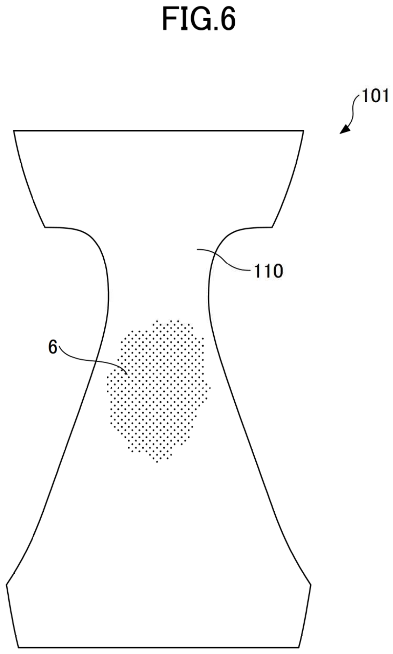

FIG. 6 is a diagram illustrating the non-skin-facing side after absorption of the menstrual blood according to Comparative Example 1.

As illustrated in FIG. 6, the sheet included in an outer body 110 of a pair of menstrual panties 101 according to Comparative Example 1 is white, such that the color seen from the non-skin-facing side before the menstrual blood absorption is white. Therefore, after blood absorption, the blood of the menstrual blood is visible from the outside of the outer body, and the color of a menstrual blood portion 6 appears significantly different from the color of other areas. Thus, the blood of the menstrual blood is visible from the outside of the outer body in Comparative Example 1.

In contrast to this, in Example 1, the L* value, which indicates the lightness of the color on the non-skin-facing side, is 19.19 before blood absorption, but 17.74 after blood absorption, and does not change much. That is, the color seen from the non-skin-facing side has not changed much from the black color seen from the non-skin-facing side before blood absorption. This is because since the color seen from the non-skin-facing side before blood absorption is black, it is difficult to see the blood of the menstrual blood through the outside of the outer body, such that the color does not change much.

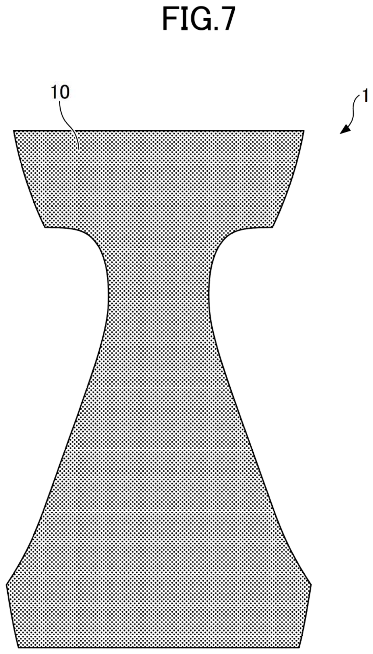

FIG. 7 is a diagram illustrating the non-skin-facing side after absorption of menstrual blood according to Example 1.

As illustrated in FIG. 7, the sheet included in the outer body 10 of the menstrual panties 1 in Example 1 is black, such that the color seen from the non-skin-facing side before blood absorption is black. Therefore, the blood of the menstrual blood is difficult to be seen through from the outside of the outer body 10 even after blood absorption.

Also, in Example 2, the L* value, which indicates the lightness of the color on the non-skin-facing side, is 37.70 before blood absorption, but is 37.25 after blood absorption. That is, the color seen from the non-skin-facing side does not change much from the dark gray color before blood absorption. This is because the color before blood absorption is dark gray as seen from the non-skin-facing side, which makes it difficult to see the blood of the menstrual blood through from the outside of the outer body, such that there is little change in color.

Thus, in Example 2, by making the sheet included in the outer body 10 dark gray, the color before blood absorption is black as seen from the non-skin-facing side, which makes it difficult to see the blood of the menstrual blood through from the outside of the outer body 10 even after blood absorption.

Thus, in the menstrual panties 1 as illustrated in FIG. 2, by making the color of the outer body 10 black or dark gray, the blood is not appreciably seen through from the outside after blood absorption. In this case, when the L* value of the outer body 10 when viewed from the non-skin-facing side of the menstrual panties 1 is 40 or less, the blood is hard to see through from the outside after blood absorption. Note that both the outer layer sheet 11 and the inner layer sheet 12 included in the outer body 10 may be made in a dark color such as black or dark gray, or one of the outer layer sheet 11 or the inner layer sheet 12 may be made in a dark color such as black or dark gray. In addition, when the L* value of the outer body 10 when viewed from the non-skin-facing side of the menstrual panties 1 is 40 or less, the gather sheet 22 and the waterproof sheet 27 included in the inner body 20 may be made in a dark color. Note that when one of the outer layer sheet 11 or the inner layer sheet 12 is made in a dark color such as black or dark gray, even when the other of the outer layer sheet 11 or the inner layer sheet 12 has a high L* value, a resultant L* value of the outer body 10 can be reduced by overlapping the outer layer sheet 11 and the inner layer sheet 12.

In addition, when the color of the outer body 10 is black or dark gray, it is less likely to give the feeling of wearing a diaper compared to white or pink, and it can give an aesthetic appearance and a feeling of underwear. In addition, it is not necessary to increase the thickness in order to prevent blood from being visible through from the outside after absorption of the menstrual blood, and it is possible to give ease of movement and slim feeling.

Furthermore, the difference at least in the L* value among the L* value, the a* value and the b* value, between the area overlapping with the inner body 20 of the outer body 10 and the area not overlapping with the inner body 20 when viewed from the non-skin-facing side of the menstrual panties 1 may be 10 or less. That is, the difference in the L* value in the CIE 1976 L*a*b* color space between the darkest area and the lightest area when viewed from the non-skin-facing side of the menstrual panties 1 may be 10 or less. In this case, when the menstrual panties 1 are viewed from the outside, which is the non-skin-facing side, it is difficult to see that the inner body 20 is arranged inside, and thus a poor appearance of the panties as the menstrual panties can be avoided.

In addition, in order to make the blood less visible from the outside of the panties after absorption of the menstrual blood, the a* value and b* value of the outer body 10 when viewed from the non-skin-facing side of the menstrual panties 1 may also be considered. The menstrual blood oxidizes over time and changes to a dull color. Therefore, if considering the a* value and b* value of the outer body 10 when the menstrual panties 1 are viewed from the outside, the blood will be more difficult to see through from the outside of the panties after the menstrual blood absorption by setting the a* value and b* value to -50 or more and 50 or less.

In a structure in which both the outer layer sheet 11 and the inner layer sheet 12 are divided into two in the front-rear direction D1, the waist portion of the present invention may include the outer body 10 and the crotch portion of the present invention may include the inner body 20. In this case, when the L* value of the sheet at the extreme of the non-skin-facing side of the inner body 20 is set to 40 or less, as in the case of the outer body 10 described above, the blood will be more difficult to be seen through from the outside of the panties after the menstrual blood absorption.

Here, if the entire menstrual panties 1 are made in a dark color such as black or dark gray, it will be difficult to check the amount and color of menstrual blood after the menstrual blood absorption.

In Example 1, the L* value indicating the lightness of the color on the skin-facing side greatly changes from 91.53 before the menstrual blood absorption to 28.47 after the menstrual blood absorption. In Example 2, the L* value indicating the lightness of the color on the skin-facing side greatly changes from 91.53 before the menstrual blood absorption to 29.57 after the menstrual blood absorption. This is because, for example, the amount of color change due to the menstrual blood absorption increases by making the top sheet 25 included in the inner body 20 white.

Thus, in the menstrual panties 1 as illustrated in FIG. 2, by making the color of the inner body 20 a light color such as white, the amount and color of the menstrual blood after the menstrual blood absorption can be readily checked. At this time, if the L* value of the inner body 20 when viewed from the skin-facing side of the menstrual panties 1 is 85 or more, the amount and color of the menstrual blood can be readily checked after the menstrual blood absorption, and a sense of cleanliness can be given.

Here, the three-dimensional gather portions 21a and 21b stand up toward the inside of the menstrual panties 1 by utilizing the contraction force of the stretchable member 24. Therefore, when the menstrual panties 1 are worn, the three-dimensional gather portions 21a and 21b may bend outward and protrude from the outer body 10.

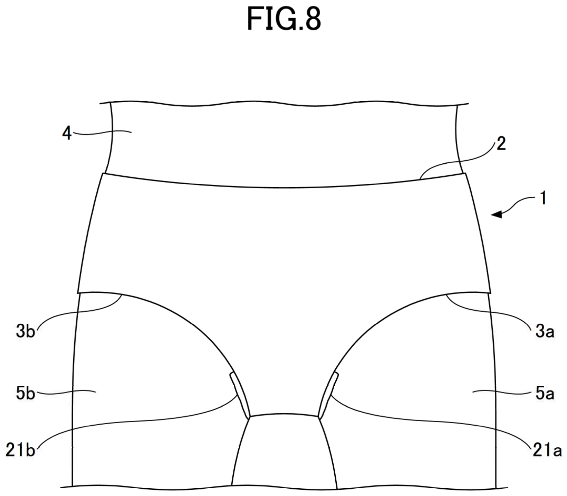

FIG. 8 is a diagram illustrating the menstrual panties 1 as illustrated in FIG. 2, in a state in which the three-dimensional gather portions 21a and 21b protrude from the outer body 10.

Since the three-dimensional gather portions 21a and 21b are configured to stand up toward the inside of the menstrual panties 1, as illustrated in FIG. 8, when the menstrual panties 1 are worn, they may be bent outward and protrude from the outer body 10. In this case, as described above, if the outer body 10 is made in a dark color such as black or dark gray and the inner body 20 is made in a light color such as white, the three-dimensional gather portions 21a and 21b stand out due to the difference in color, and the appearance deteriorates.

Therefore, only the three-dimensional gather portions 21a and 21b of the inner body 20 may be made in a dark color such as black or dark gray like the outer body 10. For example, the gather sheet 22 included in the three-dimensional gather portions 21a and 21b may be made in a dark color such as black or dark gray. When the outer body 10 is made in a dark color such as black or dark gray, it is preferable that the three-dimensional gather portions 21a and 21b are made in a dark color such as black or dark gray, as long as they have a similar color as the color of the outer body 10. Similar colors mean chromatic colors having the same hue among the 10 basic hues in the Munsell color system or achromatic colors having a lightness of less than 9 in the Munsell color system.

As a result, even when the three-dimensional gather portions 21a and 21b are bent outward and protrude from the outer body 10, they are prevented from being conspicuous and from deteriorating appearance.

### <Package 40>

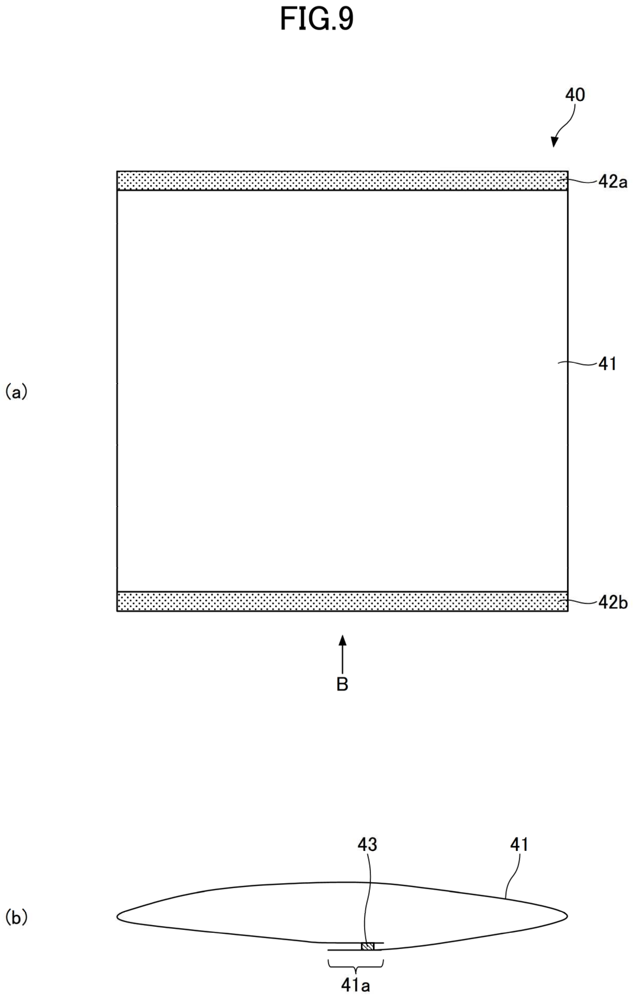

FIG. 9 is diagrams illustrating an exemplary structure of the package 40 illustrated in FIG. 1. FIG. 9(a) is a diagram illustrating a plan view of the package 40. FIG. 9(b) is a diagram illustrating a view of the package 40 seen from the direction of an arrow B illustrated in FIG. 9(a) with the package 40 opened.

As illustrated in FIG. 9, the package 40 in this example is formed of a cylindrical packaging sheet 41. In the cylindrical packaging sheet 41, the ends in the longitudinal direction overlap with each other to generate an overlapping portion 41a. The ends of the packaging sheet 41 included in the overlapping portion 41a are partially bonded together by heat, an adhesive, or the like and generate a bonding portion 43. Additionally, each end in a transverse direction of the cylindrical packaging sheet 41 is bonded to close the end by heat or the like to generate bonding portions 42a and 42b. As described above, the cylindrical packaging sheet 41 is bonded at the bonding portions 42a, 42b, and 43 to form a bag.

As the packaging sheet 41, any flexible material that can generally accommodate an article, such as a nonwoven fabric, a thin film sheet, or paper, can be used. In this case, a material having breathability or waterproof property may be used if necessary. The waterproof property may be achieved by applying a waterproof material to the surface of a material without the waterproof property. The bonding portions 42a, 42b, and 43 may be bonded at the place by applying an adhesive that melts by heating in advance and pressurizing while heating. Alternatively, for example, fine projections and recesses may be formed on paper and bonding may be achieved by meshing the projections and recesses.

A pair of menstrual panties 1 that is folded is placed on the package 40 in an unfolded state, for example, and then the package 40 is formed into a cylindrical shape to wrap the menstrual panties 1 and bonded together at the bonding portions 42a, 42b, and 43 to accommodate the menstrual panties 1.

When taking out the menstrual panties 1 from the package 40, for example, one of the bonding portion 42a or bonding portion 42b is peeled off to open the package 40 and the menstrual panties 1 are taken out from the opening.

### <Relationship between color of menstrual panties 1 and color of package 40>

The relationship between the color of the menstrual panties 1 and the color of the package 40 will be described in the following.

As described above, when the absorbent article is carried in the package body, it is preferable that the absorbent article is not visible from the outside. When disposing of an absorbent article after use, it is sometimes disposed in the package. In this case, it is not desirable that menstrual blood is visible from the outside of the package.

Therefore, the color of the material of the package 40 was changed relative to the color of the outer body 10 and the inner body 20 of the menstrual panties 1, and it was examined whether or not a pair of menstrual panties 1 could be visually recognized from the outside of the package 40 while it was enclosed in the package 40.

As described in the following, the L* value, a* value, and b* value in the CIE 1976 L*a*b* color space of the menstrual panties 1 and the L* value, a* value, and b* value of the package 40 were measured in the three cases of Examples 3 and 4 and Comparative Example 2. The value of the skin-facing side of the menstrual panties 1 is the value of the inner body when viewed from the inside of the menstrual panties 1, and the value of the non-skin-facing side of the menstrual panties 1 is the value of the outer body when viewed from the outside of the menstrual panties 1. As for the material, the values of the top sheet 25 and the outer layer sheet 11 of the menstrual panties 1 and the material of the package 40 were respectively measured.

**[Table 2]**

| | | EXAMPLE 3 | EXAMPLE 4 | COMPARATIVE EXAMPLE 2 |
|---|---|---|---|---|
| MENSTRUAL PANTIES | SKIN-FACING SIDE | WHITE | WHITE | WHITE |
| | | L*=91.53 | L*=91.53 | L*=91.53 |
| | | a*=-0.63 | a*=-0.63 | a*=-0.63 |
| | | b*=0.12 | b*=0.12 | b*=0.12 |
| | NON-SKIN-FACING SIDE | BLACK | BLACK | BLACK |
| | | L*=19.19 | L*=19.19 | L*=19.19 |
| | | a*=0.10 | a*=0.10 | a*=0.10 |
| | | b*=1.81 | b*= 1.81 | b*=1.81 |
| | TOP SHEET | WHITE NONWOVEN FABRIC | WHITE NONWOVEN FABRIC | WHITE NONWOVEN FABRIC |
| | | 22 gsm | 22 gsm | 22 gsm |
| | | L*=95.02 | L*=95.02 | L*=95.02 |
| | | a*=-0.49 | a*=-0.49 | a*=-0.49 |
| | | b*=0.82 | b*=0.82 | b*=0.82 |
| | OUTER LAYER SHEET | BLACK | BLACK | BLACK |
| | | NONWOVEN FABRIC | NONWOVEN FABRIC | NONWOVEN FABRIC |
| MATERIAL | | 14 gsm | 14 gsm | 14 gsm |
| | | L*=38.83 | L*=38.83 | L*=38.83 |
| | | a*=0.06 | a*=0.06 | a*=0.06 |
| | | b*=2.47 | b*=2.47 | b*=2.47 |
| | PACKAGE | GRAY FILM | BLACK | WHITE |
| | | | NONWOVEN FABRIC | NONWOVEN FABRIC |
| | | 26 gsm | 14 gsm | 13 gsm |
| | | L*=32.41 | L*=38.83 | L*=95.41 |
| | | a*=3.43 | a*=0.06 | a*=-0.25 |
| | | b*=6.54 | b*=2.47 | b*=1.39 |

In the menstrual panties 1, in all of Examples 3 and 4 and Comparative Example 2, as described above, the color of the non-skin-facing side was made black such that the blood would not be visible from the outside of the menstrual panties 1 after absorption of the menstrual blood, and the color of the skin-facing side was made white such that the blood volume and color could be readily confirmed after the absorption of the menstrual blood.

At this time, the L* value of the inner body 20 when viewed from the skin-facing side was 91.53, the a* value was -0.63, and the b* value was 0.12. The L* value of the outer body 10 when viewed from the non-skin-facing side was 19.19, the a* value was 0.10, and the b* value was 1.81. To achieve these values, a white nonwoven fabric of 22 gsm was used as the top sheet 25, and a black nonwoven fabric of 14 gsm was used as the outer layer sheet 11. The white nonwoven fabric had the L* value of 95.02, the a* value of -0.49, and the b* value of 0.82. The black nonwoven fabric had the L* value of 38.83, the a* value of 0.06, and the b* value of 2.47.

These materials were used for the menstrual panties 1, and the color of the material of the package 40 was changed, and whether or not the menstrual panties 1 were visible from the outside of the package 40 was examined.

In Example 3, the package 40 was made of a gray film of 26 gsm. The L* value of the gray film material in Example 3 was 32.41, the a* value was 3.43, and the b* value was 6.54.

In Example 4, the package 40 was made of black nonwoven fabric of 14 gsm. The L* value of the black nonwoven fabric material in Example 4 was 38.83, the a* value was 0.06, and the b* value was 2.47.

In Comparative Example 2, a package made of a white nonwoven fabric of 13 gsm was used. The L* value of the white nonwoven fabric material in Comparative Example 2 was 95.41, the a* value was - 0.25, and the b* value was 1.39.

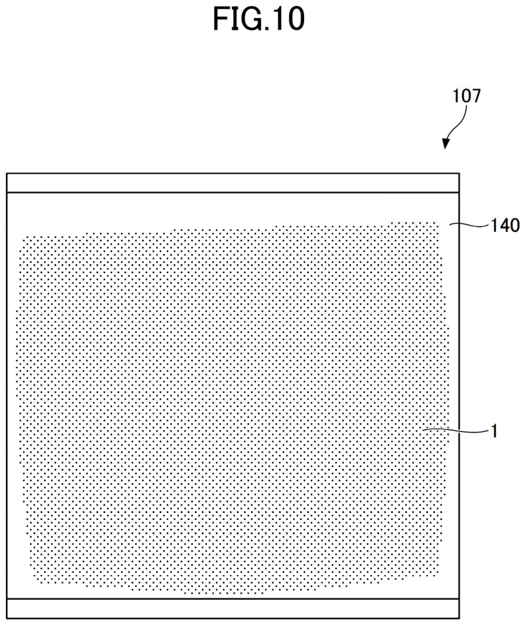

FIG. 10 is a diagram illustrating a state in which the pair of the menstrual panties 1 is enclosed in a package according to Comparative Example 2.

In an individually packaged absorbent article 107 in Comparative Example 2, the outer layer sheet 11 of the menstrual panties 1 was made of a black nonwoven fabric such that the non-skin-facing side was also black, and a package 140 was made of a white nonwoven fabric. Therefore, as illustrated in FIG. 10, the menstrual panties 1 were visible from the outside in the state where they are enclosed in the package 140.

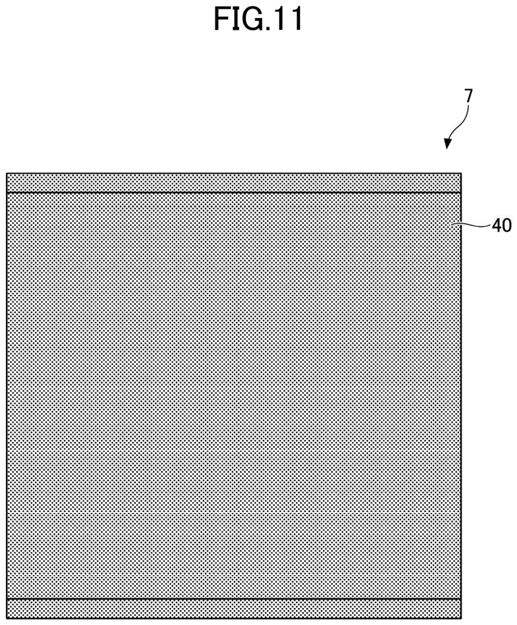

FIG. 11 is a diagram illustrating a state in which a pair of menstrual panties 1 is enclosed in the package in Example 4.

In the individually packaged absorbent article 7 in Example 4, the outer layer sheet 11 of the menstrual panties 1 was made of a black nonwoven fabric such that the non-skin-facing side was also black, and the package 40 was made of a black nonwoven fabric. Therefore, as illustrated in FIG. 11, the menstrual panties 1 cannot be readily seen from the outside in a state where they are enclosed in the package 40.

Thus, by making the color of the package 40 enclosing the menstrual panties 1 of which the color of the outer body 10 is black, the menstrual panties 1 cannot be readily seen from the outside in the state where they are enclosed in the package 40. In this case, if the L* value when the package 40 is viewed from the outside is 50 or less, it is possible to make the menstrual panties 1 enclosed in a package 40 to be not appreciably seen through from the outside of the package 40, when the L* value of the menstrual panties 1, in which the color of the outer body 10 is black or dark gray, is 40 or less. Therefore, in Example 3, since the package 40 includes a gray film having the L* value of 32.41, it is possible to make the menstrual panties 1 enclosed in a package 40 to be not appreciably seen through from the outside of the package 40, when the L* value of the menstrual panties 1, in which the color of the outer body 10 is black or dark gray, is 40 or less. The same effect can be also obtained when the L* value condition described above is satisfied in a part of the outer body 10 or the package 40. However, by satisfying the L* value condition described above in any area of the outer body 10 or the package 40, blood is not appreciably seen from the outside of the menstrual panties 1 in any area of the menstrual panties 1, and the menstrual panties 1 enclosed in a package 40 are not appreciably seen from the outside the package 40 in any area of the package 40.

Even in the menstrual panties 1 in which the crotch portion of the present invention includes the inner body 20 and the L* value of the sheet at the extreme of the non-skin-facing side of the inner body 20 is 40 or less, it is possible to make it difficult to see the menstrual panties 1 through from the outside of the package 40 when the L* value of the package 40 when viewed from the outside is 50 or less.

In addition, when the color of the package 40 in a state where the menstrual panties 1 are not enclosed in the package 40 and the color of the outer body 10 in a state where the menstrual panties 1 are not enclosed in a package 40 are made to be the same color, it is possible to further make the menstrual panties 1 to be not appreciably seen from the outside of the package 40 in a state where the menstrual panties 1 are enclosed in a package 40. For example, the package 40 and the outer body 10 may be chromatic colors having the same hue among the 10 basic hues in the Munsell color system, or achromatic colors having a lightness of less than 9 in the Munsell color system. In addition, the package 40 and the outer body 10 may be made to be the same color by selecting a material in which the difference in the a* value and the b* value between the package 40 and the outer body 10 when viewed from the outside, respectively, is 20 or less.

Although the present invention has been described based on the embodiments described above, the present invention is not limited by these embodiments. The above embodiments can be changed, modified, replaced, added, deleted, combined, and the like in various ways within the scope of the claims, which also fall within the scope of the present invention. The above components can be combined in any way.

The present application is based on and claims priority to Japanese Patent Application No. 2023-087362 filed on May 29, 2023, the entire contents of this Japanese patent application are hereby incorporated by reference.

### REFERENCE SIGNS LIST

- 1: Menstrual panties
- 2: Waist opening
- 3a, 3b: Leg opening
- 4: Trunk
- 5a, 5b: Leg
- 6: Menstrual blood portion
- 7: Individually packaged absorbent article
- 10: Outer body
- 11: Outer layer sheet
- 12: Inner layer sheet
- 13: Elastic thread
- 14a, 14b: End
- 15a to 15d, 42a, 42b, 43: Bonding portion
- 20: Inner body
- 21a, 21b: Three-dimensional gather portion
- 22: Gather sheet
- 23a, 23b: Folded portion
- 24: Stretchable member
- 25: Top sheet
- 26: Second sheet
- 27: Waterproof sheet
- 30: Absorber
- 40: Package
- 41: Packaging sheet
- 41a: Overlapping portion
- F: Front area
- M: Middle area
- R: Rear area

## Claims

1. An individually packaged absorbent article in which an absorbent article is enclosed in a package, wherein
the absorbent article is configured to form a panty shape by including
a waist portion; and
a crotch portion in which an absorber is disposed,
the absorbent article has an L* value in an CIE 1976 L*a*b* color space of 40 or less when viewed from a non-skin-facing side, the non-skin-facing side being a side opposite to a skin-facing side of the absorbent article that faces a human body, and
the package has the L* value in the CIE 1976 L*a*b* color space of 50 or less when viewed from an outside.

2. The individually packaged absorbent article according to claim 1, wherein
a color of the package in a state that the absorbent article is not enclosed in the package is the same color as a color of the absorbent article when viewed from the non-skin-facing side in the state that the absorbent article is not enclosed in the package.

3. The individually packaged absorbent article according to claim 1, wherein
a color of the absorbent article when viewed from the non-skin-facing side and a color of the package when viewed from the outside are black or gray.

4. The individually packaged absorbent article according to claim 1, wherein
the absorbent article has the L* value in the CIE 1976 L*a*b* color space, when viewed from the skin-facing side, of 40 or less in any area of the absorbent article, and
the package has the L* value in the CIE 1976 L*a*b* color space, when viewed from the outside, of 50 or less in any area of the package.
